# EUROPEAN PATENT APPLICATION

(11) **EP 3 117 762 A1**
(43) Date of publication of application: **18.01.2017**
(21) Application number: 16157970.1
(22) Date of filing: 01.03.2016
(51) Int. Cl.: A61B 5/00, A61B 5/024

(54) **APPARATUS AND METHOD FOR MEASURING HEARTBEAT/STRESS IN MOBILE TERMINAL**

(30) Priority: 13.07.2015 KR 20150099273
(71) Applicant: LG Electronics Inc., Seoul 07336 (KR)
(72) Inventor: SHIM, Hongjo, 06772 Seoul (KR); LIM, Gukchan, 06772 Seoul (KR); LEE, Yoonwoo, 06772 Seoul (KR); KIM, Seonghyok, 06772 Seoul (KR); PARK, Mihyun, 06772 Seoul (KR); KIM, Hyunwoo, 06772 Seoul (KR)
(74) Representative: Katérle, Axel

(57) **Abstract**

A circuit in a mobile terminal for measuring heartbeat/stress. The circuit includes a photoplethysmography (PPG) sensor having first and second green light-emitting diodes (LEDs); first and second LED drivers respectively connected to the first and second green LEDS and respectively configured to drive the first and second green LEDs; and a processor configured to control the first and second LED drivers to alternately turn on the first and second green LEDs within one driving period to respectively produce first and second PPG signals, and measure a user's heartbeat/stress using the first and second PPG signals that have a corresponding signal quality equal to or greater than a predetermined threshold.

## Description

### BACKGROUND OF THE INVENTION

### Field of the Invention

This specification relates to a measurement of biometric information, and more particularly, an apparatus and method for measuring heartbeat/stress in a mobile terminal, capable of consecutively measuring heartbeat/stress with low power.

### Background of the Invention

Terminals may be divided into mobile/portable terminals and stationary terminals. Also, the mobile terminals may be classified into handheld terminals and vehicle mount terminals according to whether or not a user can directly carry. Mobile terminals have become increasingly more functional. Examples of such functions include data and voice communications, capturing images and video via a camera, recording audio, playing music files via a speaker system, and displaying images and video on a display. Some mobile terminals include additional functionality which supports game playing, while other terminals are configured as multimedia players. More recently, mobile terminals have been configured to receive broadcast and multicast signals which permit viewing of content such as videos and television programs.

As it becomes multifunctional, a mobile terminal can be allowed to capture still images or moving images, play music or video files, play games, receive broadcast and the like, so as to be implemented as an integrated multimedia player. Efforts are ongoing to support and increase the functionality of mobile terminals. Such efforts include software and hardware improvements, as well as changes and improvements in the structural components.

A smart watch is a device wearable on a wrist, and is provided with various sensors for measuring user's movement and biometric information. Among others, a photoplethysmography (PPG) sensor is a heartbeat sensor which can accurately measure a quantity of user's motion, and measures heartbeats by sensing changes in light reflection along a flow of blood. Therefore, when the PPG sensor is used, a quantity of motion may further be calculated based on the measured heartbeats and provided to the user in real time, and also user's stress can be measured by analyzing changes in heartbeats.

However, when the PPG sensor is used for consecutively measuring heartbeat/stress in an always-on mode, current consumption by the PPG sensor is caused and additionally power consumption by an application processor (AP) is increased since the AP should be woken up for operation per a predetermined period of time (about 1.6 seconds) due to an interrupt signal output from the PPG sensor.

To solve these problems, a structure and method of delaying a time at which a micro controller unit (MCU) wakes the AP up is used, namely, the MCU which causes less power consumption than the AP may be provided between the PPG sensor and the AP to allow buffer sharing between the PPG sensor and the MCU. However, even though such circuit structure and method is used, a considerable amount of current is still consumed by the PPG sensor and the MCU. Furthermore, the addition of the low-power MCU causes additional costs and an increase in an installation area.

### SUMMARY OF THE INVENTION

Therefore, an aspect of the detailed description is to provide a circuit for measuring heartbeat/stress in a mobile terminal, capable of reducing current consumption caused upon an operation of a PPG sensor for a consecutive heartbeat measurement, and a method thereof.

Another aspect of the detailed description is to provide a circuit for measuring heartbeat/stress in a mobile terminal, capable of measuring heartbeat/stress using low power and the least installation area, without use of an MCU, and a method thereof.

To achieve these and other advantages and in accordance with the purpose of this specification, as embodied and broadly described herein, there is provided a circuit for measuring heartbeat/stress in a mobile terminal, the circuit including a photoplethysmography (PPG) sensor having two green light-emitting diodes (LEDs), and an application processor (AP) capable of measuring user's heartbeat/stress by analyzing PPG signals of first and second channels, detected through the two green LEDs of the PPG sensor at each driving period, wherein the two green LEDs are turned on in an alternating manner by being synchronized with each other within one driving period.

To achieve these and other advantages and in accordance with the purpose of this specification, as embodied and broadly described herein, there is provided a method for measuring heartbeat/stress in a mobile terminal, the method including detecting by a photoplethysmography (PPG) sensor PPG signals of first and second channels by operating two green light-emitting diodes (LEDs) in an alternating manner within the same driving period, transferring the detected PPG signals of the first and second channels to an application processor (AP), and measuring by the application processor heartbeat/stress by analyzing the detected PPG signals of the first and second channels.

Further scope of applicability of the present application will become more apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from the detailed description.

### BRIEF DESCRIPTION OF THE DRAWINGS

The accompanying drawings, which are included to provide a further understanding of the invention and are incorporated in and constitute a part of this specification, illustrate embodiments and together with the description serve to explain the principles of the invention.

In the drawings:
FIG. 1A is a block diagram of a mobile terminal in accordance with one embodiment of the present invention;
FIGS. 1B and 1C are conceptual views illustrating one example of the mobile terminal, viewed from different directions;
FIG. 2 is a schematic view of a general heartbeat/stress measuring circuit of a mobile terminal;
FIGS. 3A and 3B are views illustrating a buffer sharing operation using a low-power MCU;
FIG. 4 is a circuitry view of a light-emitting unit of a PPG sensor;
FIG. 5A is a view of a general connection structure of two green light-emitting diodes (LEDs);
FIG. 5B is a view illustrating an on/off operation of the two green LEDs during a heartbeat measurement;
FIG. 6 is a view illustrating an operation of sensing PPG signals through a photodiode after emitting green light from the two green LEDs;
FIG. 7 is a block diagram of a heartbeat/stress measuring circuit in a mobile terminal in accordance with an embodiment of the present invention;
FIG. 8 is a view of a connection structure of two green LEDs of a PPG sensor in accordance with an embodiment of the present invention;
FIG. 9 is a view illustrating an on/off operation of the two green LEDs in accordance with the present invention;
FIG. 10 is a view illustrating an operation of sensing PPG signals by emitting green light from the two green LEDs in accordance with an embodiment of the present invention;
FIG. 11 is a view illustrating buffer sharing between a PPG sensor and an AP in accordance with the present invention;
FIG. 12 is a view illustrating one example in which an abnormal signal is generated when measuring heartbeats using a smart watch with a PPG sensor;
FIG. 13 is a flowchart illustrating sequential steps of adaptively controlling LEDs of a PPG sensor according to qualities of PPG signals in accordance with one embodiment of the present invention;
FIG. 14 is a flowchart illustrating sequential steps of adaptively controlling LEDs of a PPG sensor according to qualities of PPG signals in accordance with another embodiment of the present invention;
FIG. 15 is a view illustrating one example of generating one PPG signal by synthesizing PPG signals of a first/second channel with bad signals;
FIG. 16 is a graph illustrating levels of PPG signals according to a skin color and an amount of hair; and
FIG. 17 is a flowchart illustrating sequential steps of adaptively controlling intensities of light emitted from LEDs of a PPG sensor according to a skin color of a wrist and an amount of hair thereon.

### DETAILED DESCRIPTION OF THE INVENTION

Description will now be given in detail according to embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In the present disclosure, that which is well-known to one of ordinary skill in the relevant art has generally been omitted for the sake of brevity. The accompanying drawings are used to help easily understand various technical features and it should be understood that the embodiments presented herein are not limited by the accompanying drawings. As such, the present disclosure should be construed to extend to any alterations, equivalents and substitutes in addition to those which are particularly set out in the accompanying drawings.

Although the terms first, second, etc. may be used herein to describe various elements, these elements should not be limited by these terms. These terms are generally only used to distinguish one element from another. When an element is referred to as being "connected with" another element, the element can be connected with the other element or intervening elements may also be present. In contrast, when an element is referred to as being "directly connected with" another element, there are no intervening elements present.

A singular representation may include a plural representation unless it represents a definitely different meaning from the context. Terms such as "include" or "has" are used herein and should be understood that they are intended to indicate an existence of several components, functions or steps, disclosed in the specification, and it is also understood that greater or fewer components, functions, or steps may likewise be utilized.

Mobile terminals presented herein may be implemented using a variety of different types of terminals. Examples of such terminals include cellular phones, smart phones, user equipment, laptop computers, digital broadcast terminals, personal digital assistants (PDAs), portable multimedia players (PMPs), navigators, portable computers (PCs), slate PCs, tablet PCs, ultra books, wearable devices (for example, smart watches, smart glasses, head mounted displays (HMDs)), and the like.

By way of non-limiting example only, further description will be made with reference to particular types of mobile terminals. However, such teachings apply equally to other types of terminals, such as those types noted above. In addition, these teachings may also be applied to stationary terminals such as digital TV, desktop computers, and the like.

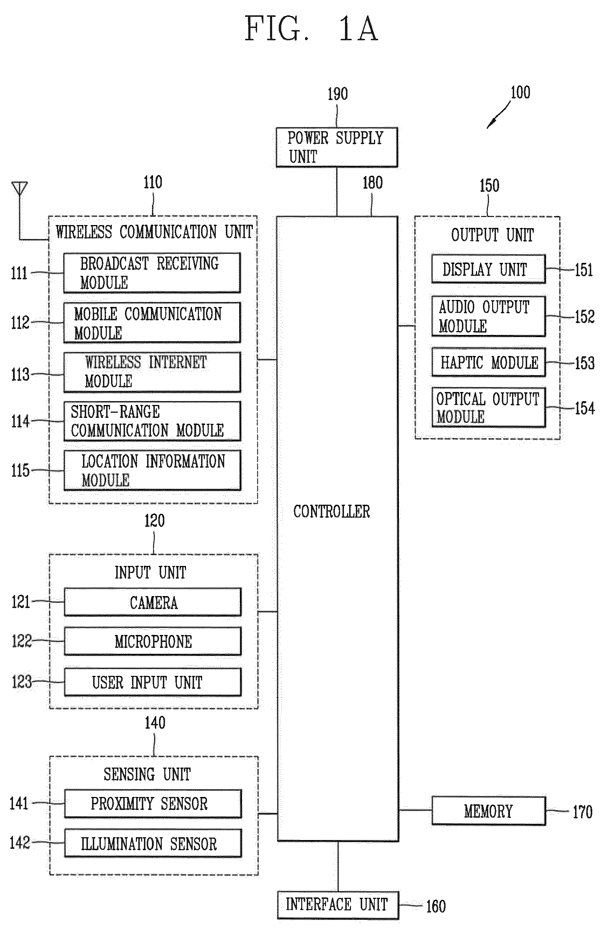

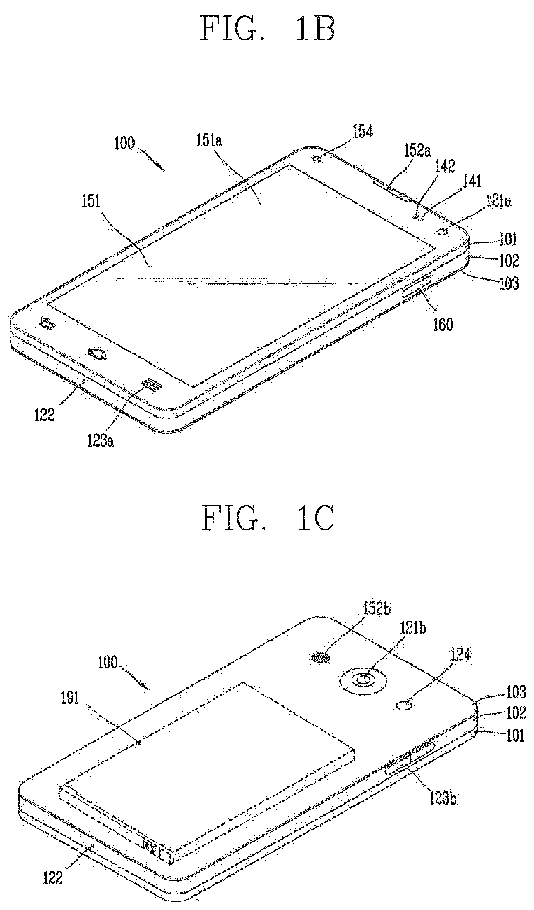

Referring to FIGS. 1A to 1C, FIG. 1A is a block diagram of a mobile terminal in accordance with one embodiment of the present invention, and FIGS. 1B and 1C are conceptual views illustrating one example of a mobile terminal, viewed from different directions.

The mobile terminal 100 may be shown having components such as a wireless communication unit 110, an input unit 120, a sensing unit 140, an output unit 150, an interface unit 160, a memory 170, a controller 180, and a power supply unit 190.Implementing all of the illustrated components is not a requirement, and that greater or fewer components may alternatively be implemented.

In more detail, the wireless communication unit 110 may typically include one or more modules which permit communications such as wireless communications between the mobile terminal 100 and a wireless communication system, communications between the mobile terminal 100 and another mobile terminal, communications between the mobile terminal 100 and an external server. Further, the wireless communication unit 110 may typically include one or more modules which connect the mobile terminal 100 to one or more networks.

The wireless communication unit 110 may include one or more of a broadcast receiving module 111, a mobile communication module 112, a wireless Internet module 113, a short-range communication module 114, and a location information module 115.

The input unit 120 may include a camera 121 or an image input unit for obtaining images or video, a microphone 122, which is one type of audio input device for inputting an audio signal, and a user input unit 123 (for example, a touch key, a mechanical key, and the like) for allowing a user to input information. Data (for example, audio, video, image, and the like) may be obtained by the input unit 120 and may be analyzed and processed according to user commands.

The sensing unit 140 may typically be implemented using one or more sensors configured to sense internal information of the mobile terminal, the surrounding environment of the mobile terminal, user information, and the like. For example, the sensing unit 140 may include at least one of a proximity sensor 141, an illumination sensor 142, a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like). The mobile terminal disclosed herein may be configured to utilize information obtained from one or more sensors of the sensing unit 140, and combinations thereof.

The output unit 150 may typically be configured to output various types of information, such as audio, video, tactile output, and the like. The output unit 150 may be shown having at least one of a display unit 151, an audio output module 152, a haptic module 153, and an optical output module 154. The display unit 151 may have an inter-layered structure or an integrated structure with a touch sensor in order to facilitate a touch screen. The touch screen may provide an output interface between the mobile terminal 100 and a user, as well as function as the user input unit 123 which provides an input interface between the mobile terminal 100 and the user.

The interface unit 160 serves as an interface with various types of external devices that can be coupled to the mobile terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the mobile terminal 100 may perform assorted control functions associated with a connected external device, in response to the external device being connected to the interface unit 160.

The memory 170 is typically implemented to store data to support various functions or features of the mobile terminal 100. For instance, the memory 170 may be configured to store application programs executed in the mobile terminal 100, data or instructions for operations of the mobile terminal 100, and the like. Some of these application programs may be downloaded from an external server via wireless communication. Other application programs may be installed within the mobile terminal 100 at time of manufacturing or shipping, which is typically the case for basic functions of the mobile terminal 100 (for example, receiving a call, placing a call, receiving a message, sending a message, and the like). It is common for application programs to be stored in the memory 170, installed in the mobile terminal 100, and executed by the controller 180 to perform an operation (or function) for the mobile terminal 100.

The controller 180 typically functions to control overall operation of the mobile terminal 100, in addition to the operations associated with the application programs. The controller 180 can provide or process information or functions appropriate for a user by processing signals, data, information and the like, which are input or output by the aforementioned various components, or activating application programs stored in the memory 170.

Also, the controller 180 controls some or all of the components illustrated in FIG. 1A according to the execution of an application program that have been stored in the memory 170. In addition, the controller 180 can control at least two of those components included in the mobile terminal to activate the application program.

The power supply unit 190 can be configured to receive external power or provide internal power in order to supply appropriate power required for operating elements and components included in the mobile terminal 100. The power supply unit 190 may include a battery, and the battery may be configured to be embedded in the terminal body, or configured to be detachable from the terminal body.

At least part of the components may cooperatively operate to implement an operation, a control or a control method of a mobile terminal according to various embodiments disclosed herein. Also, the operation, the control or the control method of the mobile terminal may be implemented on the mobile terminal by an activation of at least one application program stored in the memory 170.

Hereinafter, description will be given in more detail of the aforementioned components with reference to FIG. 1A, prior to describing various embodiments implemented through the mobile terminal 100.

First, regarding the wireless communication unit 110, the broadcast receiving module 111 is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules 111 may be utilized to facilitate simultaneously receiving of two or more broadcast channels, or to support switching among broadcast channels.

The mobile communication module 112 can transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000 (Code Division Multi Access 2000), Wideband CDMA (WCDMA), High Speed Downlink Packet access (HSDPA), High Speed Uplink Packet Access (HSUPA), Long Term Evolution (LTE), LTE-advanced (LTE-A) and the like).

Examples of the wireless signals include audio call signals, video (telephony) call signals, or various formats of data to support communication of text and multimedia messages.

The wireless Internet module 113 is configured to facilitate wireless Internet access. This module may be internally or externally coupled to the mobile terminal 100. The wireless Internet module 113 may transmit and/or receive wireless signals via communication networks according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), High Speed Uplink Packet Access (HSUPA), Long Term Evolution (LTE), LTE-advanced (LTE-A) and the like. The wireless Internet module 113 may transmit/receive data according to one or more of such wireless Internet technologies, and other Internet technologies as well.

In some embodiments, when the wireless Internet access is implemented according to, for example, WiBro, HSDPA, HSUPA, GSM, CDMA, WCDMA, LTE, LET-A, and the like, as part of a mobile communication network, the wireless Internet module 113 performs such wireless Internet access.

The short-range communication module 114 is configured to facilitate short-range communications. Suitable technologies for implementing such short-range communications include BLUETOOTH™, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB (Wireless Universal Serial Bus), and the like. The short-range communication module 114 in general supports wireless communications between the mobile terminal 100 and a wireless communication system, communications between the mobile terminal 100 and another mobile terminal 100, or communications between the mobile terminal and a network where another mobile terminal 100 (or an external server) is located, via wireless area networks. One example of the wireless area networks is a wireless personal area networks.

Here, another mobile terminal (which may be configured similarly to mobile terminal 100) may be a wearable device, for example, a smart watch, a smart glass or a head mounted display (HMD), which can exchange data with the mobile terminal 100 (or otherwise cooperate with the mobile terminal 100). The short-range communication module 114 may sense or recognize the wearable device, and permit communication between the wearable device and the mobile terminal 100. In addition, when the sensed wearable device is a device which is authenticated to communicate with the mobile terminal 100, the controller 180, for example, may cause transmission of at least part of data processed in the mobile terminal 100 to the wearable device via the short-range communication module 114. Hence, a user of the wearable device may use the data processed in the mobile terminal 100 on the wearable device. For example, when a call is received in the mobile terminal 100, the user may answer the call using the wearable device. Also, when a message is received in the mobile terminal 100, the user can check the received message using the wearable device.

The location information module 115 is generally configured to detect, calculate, derive or otherwise identify a position (or current position) of the mobile terminal. As an example, the location information module 115 includes a Global Position System (GPS) module, a Wi-Fi module, or both. For example, when the mobile terminal uses a GPS module, a position of the mobile terminal may be acquired using a signal sent from a GPS satellite. As another example, when the mobile terminal uses the Wi-Fi module, a position of the mobile terminal can be acquired based on information related to a wireless access point (AP) which transmits or receives a wireless signal to or from the Wi-Fi module. If desired, the location information module 115 may alternatively or additionally function with any of the other modules of the wireless communication unit 110 to obtain data related to the position of the mobile terminal. The location information module 115 is a module used for acquiring the position (or the current position) and may not be limited to a module for directly calculating or acquiring the position of the mobile terminal.

The input unit 120 may be configured to permit various types of inputs to the mobile terminal 120. Examples of such inputs include audio, image, video, data, and user input. Image and video input is often obtained using one or more cameras 121. Such cameras 121 may process image frames of still pictures or video obtained by image sensors in a video or image capture mode. The processed image frames can be displayed on the display unit 151 or stored in memory 170. Meanwhile, the cameras 121 may be arranged in a matrix configuration to permit a plurality of images having various angles or focal points to be input to the mobile terminal 100. Also, the cameras 121 may be located in a stereoscopic arrangement to acquire left and right images for implementing a stereoscopic image.

The microphone 122 processes an external audio signal into electric audio (sound) data. The processed audio data can be processed in various manners according to a function being executed in the mobile terminal 100. If desired, the microphone 122 may include assorted noise removing algorithms to remove unwanted noise generated in the course of receiving the external audio signal.

The user input unit 123 is a component that permits input by a user. Such user input may enable the controller 180 to control operation of the mobile terminal 100. The user input unit 123 may include one or more of a mechanical input element (for example, a mechanical key, a button located on a front and/or rear surface or a side surface of the mobile terminal 100, a dome switch, a jog wheel, a jog switch, and the like), or a touch-sensitive input element, among others. As one example, the touch-sensitive input element may be a virtual key, a soft key or a visual key, which is displayed on a touch screen through software processing, or a touch key which is located on the mobile terminal at a location that is other than the touch screen. Further, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like, and generate a corresponding sensing signal. The controller 180 generally cooperates with the sending unit 140 to control operation of the mobile terminal 100 or execute data processing, a function or an operation associated with an application program installed in the mobile terminal based on the sensing signal. The sensing unit 140 may be implemented using any of a variety of sensors, some of which will now be described in more detail.

The proximity sensor 141 refers to a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this instance, the touch screen (touch sensor) may also be categorized as a proximity sensor.

The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like). In general, controller 180 processes data corresponding to proximity touches and proximity touch patterns sensed by the proximity sensor 141, and cause output of visual information on the touch screen. In addition, the controller 180 can control the mobile terminal 100 to execute different operations or process different data (or information) according to whether a touch with respect to a point on the touch screen is either a proximity touch or a contact touch.

A touch sensor can sense a touch (or a touch input) applied to the touch screen, such as display unit 151, using any of a variety of touch methods. Examples of such touch methods include a resistive type, a capacitive type, an infrared type, and a magnetic field type, among others.

As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched area, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, corresponding signals may be transmitted to a touch controller. The touch controller may process the received signals, and then transmit corresponding data to the controller 180. Accordingly, the controller 180 can sense which region of the display unit 151 has been touched. Here, the touch controller may be a component separate from the controller 180, the controller 180, and combinations thereof.

Meanwhile, the controller 180 can execute the same or different controls according to a type of touch object that touches the touch screen or a touch key provided in addition to the touch screen. Whether to execute the same or different control according to the object which provides a touch input may be decided based on a current operating state of the mobile terminal 100 or a currently executed application program, for example.

The touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

If desired, an ultrasonic sensor may be implemented to recognize location information relating to a touch object using ultrasonic waves. The controller 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121, which has been depicted as a component of the input unit 120, typically includes at least one a camera sensor (CCD, CMOS etc.), a photo sensor (or image sensors), and a laser sensor. Implementing the camera 121 with a laser sensor may allow detection of a touch of a physical object with respect to a 3D stereoscopic image. The photo sensor may be laminated on, or overlapped with, the display device. The photo sensor may be configured to scan movement of the physical object in proximity to the touch screen. In more detail, the photo sensor may include photo diodes and transistors at rows and columns to scan content received at the photo sensor using an electrical signal which changes according to the quantity of applied light. Namely, the photo sensor may calculate the coordinates of the physical object according to variation of light to thus obtain location information of the physical object.

The display unit 151 is generally configured to output information processed in the mobile terminal 100. For example, the display unit 151 may display execution screen information of an application program executing at the mobile terminal 100 or user interface (UI) and graphic user interface (GUI) information in response to the execution screen information.

Also, the display unit 151 may be implemented as a stereoscopic display unit for displaying stereoscopic images. A typical stereoscopic display unit may employ a stereoscopic display scheme such as a stereoscopic scheme (a glass scheme), an auto-stereoscopic scheme (glassless scheme), a projection scheme (holographic scheme), or the like.

The audio output module 152 is generally configured to output audio data. Such audio data may be obtained from any of a number of different sources, such that the audio data may be received from the wireless communication unit 110 or may have been stored in the memory 170. The audio data may be output during modes such as a signal reception mode, a call mode, a record mode, a voice recognition mode, a broadcast reception mode, and the like. The audio output module 152 can provide audible output related to a particular function (e.g., a call signal reception sound, a message reception sound, etc.) performed by the mobile terminal 100. The audio output module 152 may also be implemented as a receiver, a speaker, a buzzer, or the like.

A haptic module 153 can be configured to generate various tactile effects that a user feels, perceive, or otherwise experience. A typical example of a tactile effect generated by the haptic module 153 is vibration. The strength, pattern and the like of the vibration generated by the haptic module 153 can be controlled by user selection or setting by the controller. For example, the haptic module 153 may output different vibrations in a combining manner or a sequential manner.

Besides vibration, the haptic module 153 can generate various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that can absorb or generate heat, and the like.

The haptic module 153 can also be implemented to allow the user to feel a tactile effect through a muscle sensation such as the user's fingers or arm, as well as transferring the tactile effect through direct contact. Two or more haptic modules 153 may be provided according to the particular configuration of the mobile terminal 100.

An optical output module 154 can output a signal for indicating an event generation using light of a light source. Examples of events generated in the mobile terminal 100 may include message reception, call signal reception, a missed call, an alarm, a schedule notice, an email reception, information reception through an application, and the like.

A signal output by the optical output module 154 may be implemented so the mobile terminal emits monochromatic light or light with a plurality of colors. The signal output may be terminated as the mobile terminal senses that a user has checked the generated event, for example.

The interface unit 160 serves as an interface for external devices to be connected with the mobile terminal 100. For example, the interface unit 160 can receive data transmitted from an external device, receive power to transfer to elements and components within the mobile terminal 100, or transmit internal data of the mobile terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The identification module may be a chip that stores various information for authenticating authority of using the mobile terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device can be connected with the terminal 100 via the interface unit 160.

When the mobile terminal 100 is connected with an external cradle, the interface unit 160 can serve as a passage to allow power from the cradle to be supplied to the mobile terminal 100 or may serve as a passage to allow various command signals input by the user from the cradle to be transferred to the mobile terminal there through. Various command signals or power input from the cradle may operate as signals for recognizing that the mobile terminal is properly mounted on the cradle.

The memory 170 can store programs to support operations of the controller 180 and store input/output data (for example, phonebook, messages, still images, videos, etc.). The memory 170 may store data related to various patterns of vibrations and audio which are output in response to touch inputs on the touch screen.

The memory 170 may include one or more types of storage mediums including a flash memory type, a hard disk type, a solid state disk (SSD) type, a silicon disk drive (SDD) type, a multimedia card micro type, a card-type memory (e.g., SD or DX memory, etc.), a Random Access Memory (RAM), a Static Random Access Memory (SRAM), a Read-Only Memory (ROM), an Electrically Erasable Programmable Read-Only Memory (EEPROM), a Programmable Read-Only memory (PROM), a magnetic memory, a magnetic disk, an optical disk, and the like. The mobile terminal 100 may also be operated in relation to a network storage device that performs the storage function of the memory 170 over a network, such as the Internet.

The controller 180 can typically control operations relating to application programs and the general operations of the mobile terminal 100. For example, the controller 180 can set or release a lock state for restricting a user from inputting a control command with respect to applications when a status of the mobile terminal meets a preset condition.

The controller 180 can also perform the controlling and processing associated with voice calls, data communications, video calls, and the like, or perform pattern recognition processing to recognize a handwriting input or a picture drawing input performed on the touch screen as characters or images, respectively. In addition, the controller 180 can control one or a combination of those components in order to implement various embodiments disclosed herein.

The power supply unit 190 receives external power or provide internal power and supply the appropriate power required for operating respective elements and components included in the mobile terminal 100. The power supply unit 190 may include a battery, which is typically rechargeable or be detachably coupled to the terminal body for charging.

The power supply unit 190 may include a connection port. The connection port may be configured as one example of the interface unit 160 to which an external charger for supplying power to recharge the battery is electrically connected.

As another example, the power supply unit 190 may be configured to recharge the battery in a wireless manner without use of the connection port. In this example, the power supply unit 190 can receive power, transferred from an external wireless power transmitter, using at least one of an inductive coupling method which is based on magnetic induction or a magnetic resonance coupling method which is based on electromagnetic resonance. Various embodiments described herein may be implemented in a computer-readable medium, a machine-readable medium, or similar medium using, for example, software, hardware, or any combination thereof.

Referring now to FIGS. 1B and 1C, the mobile terminal 100 is described with reference to a bar-type terminal body. However, the mobile terminal 100 may alternatively be implemented in any of a variety of different configurations. Examples of such configurations include watch-type, clip-type, glasses-type, or as a folder-type, flip-type, slide-type, swing-type, and swivel-type in which two and more bodies are combined with each other in a relatively movable manner, and combinations thereof. Discussion herein will often relate to a particular type of mobile terminal. However, such teachings with regard to a particular type of mobile terminal will generally apply to other types of mobile terminals as well. Here, considering the mobile terminal 100 as at least one assembly, the terminal body may be understood as a conception referring to the assembly.

The mobile terminal 100 will generally include a case (for example, frame, housing, cover, and the like) forming the appearance of the terminal. In this embodiment, the case is formed using a front case 101 and a rear case 102. Various electronic components are incorporated into a space formed between the front case 101 and the rear case 102. At least one middle case may be additionally positioned between the front case 101 and the rear case 102.

The display unit 151 is shown located on the front side of the terminal body to output information. As illustrated, a window 151 a of the display unit 151 may be mounted to the front case 101 to form the front surface of the terminal body together with the front case 101.

In some embodiments, electronic components may also be mounted to the rear case 102. Examples of such electronic components include a detachable battery 191, an identification module, a memory card, and the like. Rear cover 103 is shown covering the electronic components, and this cover may be detachably coupled to the rear case 102. Therefore, when the rear cover 103 is detached from the rear case 102, the electronic components mounted to the rear case 102 are externally exposed.

As illustrated, when the rear cover 103 is coupled to the rear case 102, a side surface of the rear case 102 is partially exposed. In some cases, upon the coupling, the rear case 102 may also be completely shielded by the rear cover 103. In some embodiments, the rear cover 103 may include an opening for externally exposing a camera 121b or an audio output module 152b.

The cases 101, 102, 103 may be formed by injection-molding synthetic resin or may be formed of a metal, for example, stainless steel (STS), aluminum (Al), titanium (Ti), or the like. As an alternative to the example in which the plurality of cases form an inner space for accommodating components, the mobile terminal 100 may be configured such that one case forms the inner space. In this example, a mobile terminal 100 having a uni-body is formed so synthetic resin or metal extends from a side surface to a rear surface.

If desired, the mobile terminal 100 may include a waterproofing unit for preventing introduction of water into the terminal body. For example, the waterproofing unit may include a waterproofing member which is located between the window 151a and the front case 101, between the front case 101 and the rear case 102, or between the rear case 102 and the rear cover 103, to hermetically seal an inner space when those cases are coupled.

The mobile terminal 100 may include a display unit 151, first and second audio output module 152a and 152b, a proximity sensor 141, an illumination sensor 142, an optical output module 154, first and second cameras 121a and 121b, first and second manipulation units 123a and 123b, a microphone 122, an interface unit 160, and the like.

Hereinafter, as illustrated in FIGS. 1B and 1C, description will be given of the mobile terminal 100 in which the front surface of the terminal body is shown having the display unit 151, the first audio output module 152a, the proximity sensor 141, the illumination sensor 142, the optical output module 154, the first camera 121a, and the first manipulation unit 123a, the side surface of the terminal body is shown having the second manipulation unit 123b, the microphone 122, and the interface unit 160, and the rear surface of the terminal body is shown having the second audio output module 152b and the second camera 121b.

However, those components may not be limited to the arrangement. Some components may be omitted or rearranged or located on different surfaces. For example, the first manipulation unit 123a may be located on another surface of the terminal body, and the second audio output module 152b may be located on the side surface of the terminal body other than the rear surface of the terminal body.

The display unit 151 outputs information processed in the mobile terminal 100. For example, the display unit 151 may display execution screen information of an application program executing at the mobile terminal 100 or user interface (UI) and graphic user interface (GUI) information in response to the execution screen information.

The display unit 151 may be implemented using one or more suitable display devices. Examples of such suitable display devices include a liquid crystal display (LCD), a thin film transistor-liquid crystal display (TFT-LCD), an organic light emitting diode (OLED), a flexible display, a 3-dimensional (3D) display, an e-ink display, and combinations thereof.

The display unit 151 may be implemented using two display devices, which can implement the same or different display technology. For instance, a plurality of the display units 151 may be arranged on one side, either spaced apart from each other, or these devices may be integrated, or these devices may be arranged on different surfaces.

The display unit 151 may also include a touch sensor which senses a touch input received at the display unit. When a touch is input to the display unit 151, the touch sensor may be configured to sense this touch and the controller 180, for example, may generate a control command or other signal corresponding to the touch. The content which is input in the touching manner may be a text or numerical value, or a menu item which can be indicated or designated in various modes.

The touch sensor may be configured in a form of a film having a touch pattern, disposed between the window 151a and a display on a rear surface of the window 151a, or a metal wire which is patterned directly on the rear surface of the window 151a. Alternatively, the touch sensor may be integrally formed with the display. For example, the touch sensor may be disposed on a substrate of the display or within the display.

The display unit 151 may also form a touch screen together with the touch sensor. Here, the touch screen may serve as the user input unit 123 (see FIG. 1A). Therefore, the touch screen may replace at least some of the functions of the first manipulation unit 123a.

The first audio output module 152a may be implemented in the form of a receiver for transferring call sounds to a user's ear and the second audio output module 152b may be implemented in the form of a loud speaker to output alarm sounds, multimedia audio reproduction, and the like.

The window 151a of the display unit 151 will typically include an aperture to permit audio generated by the first audio output module 152a to pass. One alternative is to allow audio to be released along an assembly gap between the structural bodies (for example, a gap between the window 151a and the front case 101). In this instance, a hole independently formed to output audio sounds may not be seen or is otherwise hidden in terms of appearance, thereby further simplifying the appearance and manufacturing of the mobile terminal 100.

The optical output module 154 can be configured to output light for indicating an event generation. Examples of such events include a message reception, a call signal reception, a missed call, an alarm, a schedule alarm, an email reception, information reception through an application, and the like. When a user has checked a generated event, the controller 180 can control the optical output module 154 to stop the light output.

The first camera 121a can process image frames such as still or moving images obtained by the image sensor in a capture mode or a video call mode. The processed image frames can then be displayed on the display unit 151 or stored in the memory 170.

The first and second manipulation units 123a and 123b are examples of the user input unit 123, which may be manipulated by a user to provide input to the mobile terminal 100. The first and second manipulation units 123a and 123b may also be commonly referred to as a manipulating portion, and may employ any tactile method that allows the user to perform manipulation such as touch, push, scroll, or the like. The first and second manipulation units 123a and 123b may also employ any non-tactile method that allows the user to perform manipulation such as proximity touch, hovering, or the like.

FIG. 1B illustrates the first manipulation unit 123a as a touch key, but possible alternatives include a mechanical key, a push key, a touch key, and combinations thereof. Input received at the first and second manipulation units 123a and 123b may be used in various ways. For example, the first manipulation unit 123a may be used by the user to provide an input to a menu, home key, cancel, search, or the like, and the second manipulation unit 123b may be used by the user to provide an input to control a volume level being output from the first or second audio output modules 152a or 152b, to switch to a touch recognition mode of the display unit 151, or the like.

As another example of the user input unit 123, a rear input unit may be located on the rear surface of the terminal body. The rear input unit can be manipulated by a user to provide input to the mobile terminal 100. The input may be used in a variety of different ways. For example, the rear input unit may be used by the user to provide an input for power on/off, start, end, scroll, control volume level being output from the first or second audio output modules 152a or 152b, switch to a touch recognition mode of the display unit 151, and the like. The rear input unit may be configured to permit touch input, a push input, or combinations thereof.

The rear input unit may be located to overlap the display unit 151 of the front side in a thickness direction of the terminal body. As one example, the rear input unit may be located on an upper end portion of the rear side of the terminal body such that a user can easily manipulate it using a forefinger when the user grabs the terminal body with one hand. Alternatively, the rear input unit can be positioned at most any location of the rear side of the terminal body.

When the rear input unit is provided on the rear surface of the terminal body, new types of user interfaces using the rear input unit can be implemented. Embodiments that include the aforementioned touch screen or the rear input unit may implement some or all of the functionality of the first manipulation unit 123a provided on the front surface of the terminal body. As such, in situations where the first manipulation unit 123a is omitted from the front side, the display unit 151 can have a larger screen.

As a further alternative, the mobile terminal 100 may include a finger scan sensor which scans a user's fingerprint. The controller 180 can then use fingerprint information sensed by the finger scan sensor as part of an authentication procedure. The finger scan sensor may also be installed in the display unit 151 or implemented in the user input unit 123.

The microphone 122 is shown located at an end of the mobile terminal 100, but other locations are possible. If desired, multiple microphones may be implemented, with such an arrangement permitting the receiving of stereo sounds.

The interface unit 160 may serve as a path allowing the mobile terminal 100 to interface with external devices. For example, the interface unit 160 may include one or more of a connection terminal for connecting to another device (for example, an earphone, an external speaker, or the like), a port for near field communication (for example, an Infrared Data Association (IrDA) port, a Bluetooth port, a wireless LAN port, and the like), or a power supply terminal for supplying power to the mobile terminal 100. The interface unit 160 may be implemented in the form of a socket for accommodating an external card, such as Subscriber Identification Module (SIM), User Identity Module (UIM), or a memory card for information storage.

The second camera 121b is shown located at the rear side of the terminal body and includes an image capturing direction that is substantially opposite to the image capturing direction of the first camera unit 121a. The second camera 121b can include a plurality of lenses arranged along at least one line. The plurality of lenses may also be arranged in a matrix configuration. The cameras may be referred to as an "array camera." When the second camera 121b is implemented as an array camera, images may be captured in various manners using the plurality of lenses and images with better qualities.

As shown in FIG. 1C, a flash 124 is shown adjacent to the second camera 121b. When an image of a subject is captured with the camera 121b, the flash 124 may illuminate the subject. The second audio output module 152b can be located on the terminal body. The second audio output module 152b may implement stereophonic sound functions in conjunction with the first audio output module 152a, and may be also used for implementing a speaker phone mode for call communication.

At least one antenna for wireless communication may be located on the terminal body. The antenna may be installed in the terminal body or formed by the case. For example, an antenna which configures a part of the broadcast receiving module 111 may be retractable into the terminal body. Alternatively, an antenna may be formed using a film attached to an inner surface of the rear cover 103, or a case that includes a conductive material.

A power supply unit 190 for supplying power to the mobile terminal 100 may include a battery 191, which is mounted in the terminal body or detachably coupled to an outside of the terminal body. The battery 191 may receive power via a power source cable connected to the interface unit 160. Also, the battery 191 can be recharged in a wireless manner using a wireless charger. Wireless charging may be implemented by magnetic induction or electromagnetic resonance.

The rear cover 103 is shown coupled to the rear case 102 for shielding the battery 191, to prevent separation of the battery 191, and to protect the battery 191 from an external impact or from foreign material. When the battery 191 is detachable from the terminal body, the rear case 103 may be detachably coupled to the rear case 102.

An accessory for protecting an appearance or assisting or extending the functions of the mobile terminal 100 can also be provided on the mobile terminal 100. As one example of an accessory, a cover or pouch for covering or accommodating at least one surface of the mobile terminal 100 may be provided. The cover or pouch may cooperate with the display unit 151 to extend the function of the mobile terminal 100. Another example of the accessory is a touch pen for assisting or extending a touch input to a touch screen.

Hereinafter, description will be given of embodiments associated with a control method which can be implemented in the mobile terminal having such configuration, with reference to the accompanying drawings. It will be obvious to those skilled in the art that the present disclosure can be specified into other particular forms without departing from the spirit and essential characteristics of the present disclosure.

An always-on function may be applied to various functions of a terminal which are sensed by sensors. For example, the always-on function may be applied to smart wake-up of turning an LCD on, activity monitoring for monitoring user's activities, sleep monitoring of measuring quality of sleep, and PPG sensor monitoring of measuring heartbeat/stress.

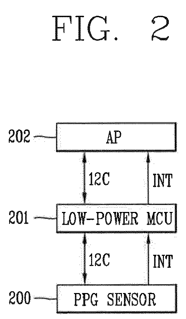

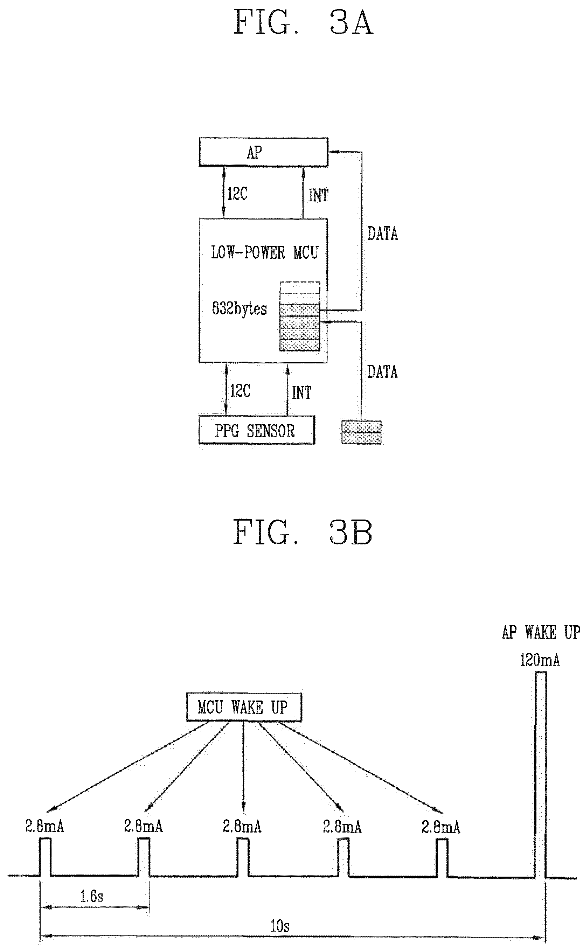

FIG. 2 is a schematic view of a general heartbeat/stress measuring circuit of a mobile terminal, and FIGS. 3A and 3B are views illustrating a buffer sharing operation using a low-power MCU. Referring to FIG. 2, a general heartbeat measuring circuit may include a PPG sensor 200 that measures an absorption ratio of green light, which is emitted to a user's body (e.g., wrist) and changed due to blood-flow, by use of a photodiode (PD), a low-power MCU 201 that wakes up at a predetermined period (e.g., about 1.6 seconds) by an interrupt signal INT output from the PPG sensor 200 and buffers measurement data of the PPG sensor 200, and an AP 202 that wakes up at a predetermined period (e.g., 10 seconds) by an interrupt signal INT output from the low-power MCU 201 and analyzes the measurement data buffered in the low-power MCU to measure heartbeats (or stress) of the user.

The AP 202 may be the controller 180 disclosed herein. The AP 202 may also have a program installed therein for measuring the heartbeat (stress), and output the measured heartbeat/stress on the display unit 151 or store the measured heartbeat/stress in the memory 170. Here, I2C denotes I2C communication which allows for transmission and reception of control signals and data.

In order to consecutively measure the heartbeat/stress by operating (or driving) the PPG sensor 200 in the always-on mode in the aforementioned circuit structure, as illustrated in FIGS. 3A and 3B, the PPG sensor 200 may wake up the low-power MCU 201 by outputting an interrupt signal INT at a period of 1.6 seconds, and stack sensed PPG signals (data) in a buffer of the low-power MCU 201. Afterwards, when the PPG signals (data) more than a threshold value are stacked in the buffer, the low-power MCU 201 can wake up the AP 202 by outputting an interrupt signal INT at a period of 10 seconds, and output the stacked PPG signals (data) to the AP 202. This may cause current consumption by the low-power MCU 201 (FIG. 3B), and an increase in additional costs and installation area for arranging the low-power MCU 201.

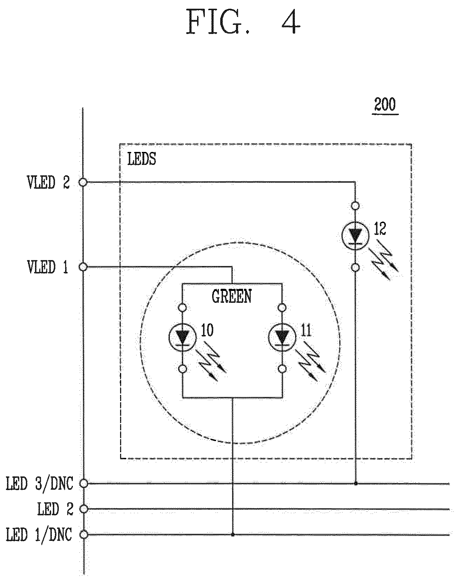

FIG. 4 is a circuitry view of a light-emitting unit of a PPG sensor. Referring to FIG. 4, a light-emitting unit of the PPG sensor 200 includes two green light-emitting diodes (LEDs) 10 and 11 for measuring heartbeats, and one infrared (IR) LED 12. The two green LEDs 10 and 11 are for measuring the heartbeats, and the one IR LED 12 is for sensing whether or not the smart watch is warn.

The two green LEDs 10 and 11 may be spaced apart from each other by a predetermined distance, and a photodiode as a light receiving unit may be interposed between the two green LEDs 10 and 11. Therefore, since two green LEDS have a wider measurement coverage than one green LED, smart watches preferably include a PPG sensor having two green LEDs.

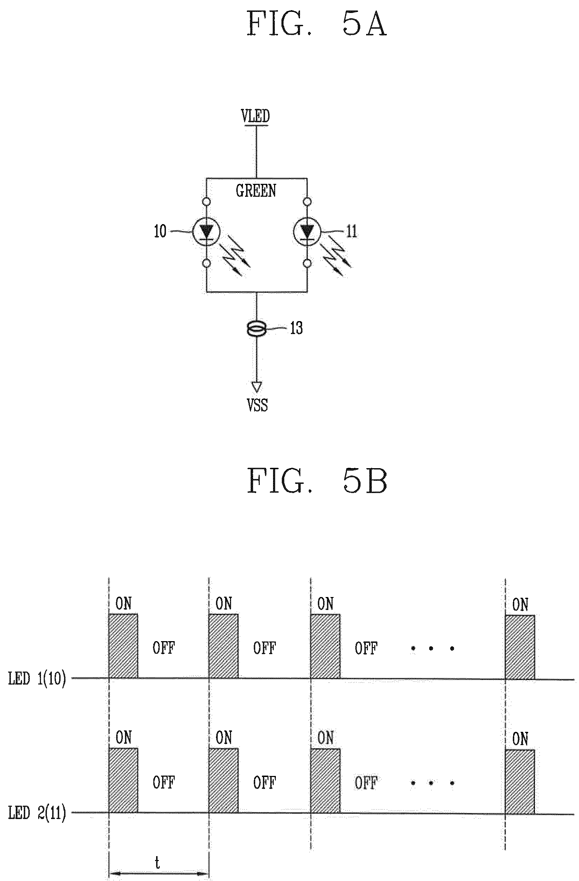

Next, FIG. 5A is a view of a general connection structure of two green LEDs, and FIG. 5B is a timing view of a driving signal for turning on/off the two green LEDs during a heartbeat measurement. Referring to FIG. 5A, the two green LEDs 10 and 11 are connected to one LED driver (or switch) 13. The LED driver 13 which is a device for turning on/off the green LEDs 10 and 12 may include a metal oxide semiconductor (MOS) transistor FET.

When a driving signal is applied to the LED driver 13, the MOS transistor FET can be turned on. Accordingly, as illustrated in FIG. 5B, the green LEDs 10 and 11 can simultaneously be turned on/off within each driving signal period (t) by a driving voltage VLED, thereby emitting green light onto a user's skin.

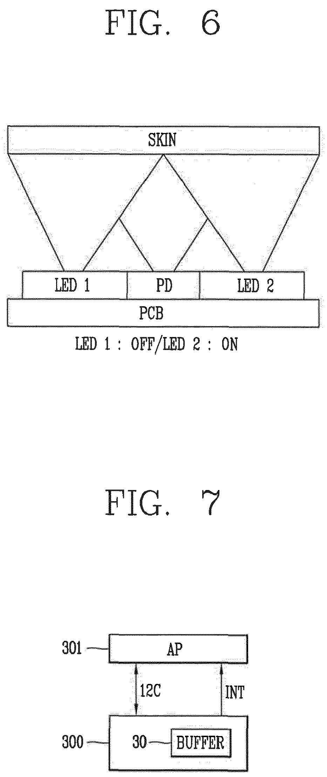

FIG. 6 is a view illustrating an operation of sensing PPG signals through a photodiode after emitting green light from the two green LEDs. As illustrated in FIG. 6, the PPG sensor 200 may include one photodiode PD interposed between the green LEDs 10 and 11 (or LED1 and LED2). When the two green LEDs 10 and 11 are simultaneously turned on by a driving signal to emit (radiate) green light to a skin, the photodiode PD can add up green light of the green LEDs 10 and 11 reflected on the skin, to generate one PPG signal. That is, the photodiode PD can generate one PPG signal per each driving signal period (t). To obtain two PPG signals at the driving signal period (t), the LEDs 10 and 11 should simultaneously be turned on twice by applying the driving signal twice.

Therefore, the method in which the two green LEDs 10 and 11 are simultaneously turned on at each driving signal period (t) has drawbacks in that a large current consumption is caused and a detection efficiency of the PPG signal is lowered.

Thus, the present invention provides an always-on heartbeat/stress measurement method capable of consecutively measuring a user's heartbeat/stress with low power by using an always-on PPG sensor, which is installed in a mobile terminal, and more particularly, a wearable device (e.g., smart watch).

The present invention takes into account both hardware and software improvements as the low-power always-on heartbeat/stress measurement method. From the perspective of the hardware improvements, the present invention provides three features, namely, non-use of a low-power MCU, a change in a connection structure between LEDs and an LED driver of a PPG sensor, and using a data buffering structure within the PPG sensor.

From the perspective of the software improvements, the present invention provides three features, namely, turn-on of two green LEDs, among the two green LEDs and one IR LED constructing the PPG sensor, in an alternating manner at the same period, turn-off of a specific LED (channel) by analyzing a signal quality of the PPG sensor, and a control of a current of the PPG sensor by analyzing a user's skin tone.

Next, FIG. 7 is a block diagram of a heartbeat/stress measuring circuit in a mobile terminal in accordance with an embodiment of the present invention. As illustrated in FIG. 7, the heartbeat measuring circuit of a mobile terminal according to an embodiment of the present invention includes a PPG sensor 300 and an AP 301. The AP 301 may correspond to the controller 180 of FIG. 1.

Further, the PPG sensor 300 may include two green LEDs for measuring heartbeats, one IR LED, and a photodiode interposed between the two green LEDs. The PPG sensor 300 may also be provided with a buffer 30, for example, first-in, first-out (FIFO), in which sensed PPG signals can be stacked by a threshold value (e.g., 832 bytes). The buffer 30 can output an interrupt signal INT to the AP 301 to wake up the AP 301 only when the stacked PPG signals reach the threshold value.

Therefore, for example, assuming the PPG sensor 300 has a stress output data rate (ODR) of 200 Hz, a heartbeat ODR of 20 Hz, and a number of sensed bytes of the PPG signal which is 4 bytes, the AP 301 is awakened 20 times per second and 4-byte heartbeat data is transferred to the AP 301 at each time during the heartbeat measurement, whereas the AP 301 is awakened 200 times per second and 4-byte stress data is transferred to the AP 301 at each time during the stress measurement, in the related art. Accordingly, during the heartbeat measurement, totally consumed current is 7.5 mA, namely, 1.2 mA by the PPG sensor 300 and 6.3 mA by the AP 301. In addition, during the stress measurement, totally consumed current is 15.9 mA, namely, 1.2 mA by the PPG sensor 300 and 14.7 mA by the AP 301.

Under the same condition, the PPG sensor 300 according to an embodiment of the present invention stacks in the FIFO heartbeat data of 80 bytes (20 Hz x 4 bytes) during the heartbeat measurement and stress data of 800 bytes (200 Hz x 4 bytes) during the stress measurement, and then transfers that data to the AP 301 at once. As a result, the PPG sensor 300 consumes 1.2 mA and the AP 301 consumes 0.6 mA during the heartbeat measurement, and thus a total amount of consumed currents is 1.8 mA.

Further, the PPG sensor 300 consumes 1.2 mA and the AP 301 consumes 0.9 mA during the stress measurement, and thus a total amount of consumed currents is 2.1 mA. In this instance, the PPG sensor 300 wakes up the AP 301 once per 10 seconds during the heartbeat measurement, and once per one second during the stress measurement.

Thus, unlike the related art in which the AP is awakened 20 times per second during the heartbeat measurement and 200 times per second during the stress measurement, even though the heartbeat measuring circuit is implemented by the PPG sensor 300 and the AP 301 in accordance with the present invention, when heartbeat data and stress data measured for one second are all stacked in the FIFO, the PPG sensor 300 may awake the AP 301 one time and transfer the stacked heartbeat and stress data to the AP 301 at once. This buffer sharing structure according to an embodiment of the present invention can arouse a remarkable reduction of currents consumed by the AP 301 during the heartbeat/stress measure.

Therefore, a time period (or time interval) at which the PPG sensor 300 awakes the AP 301 may be decided by a size (or amount) of data (heartbeat and stress data) stacked in the buffer (FIFO), namely, decided by a threshold value, and the threshold value may be decided by a heartbeat ODR, a stress ODR and a number of sensed bytes of the PPG sensor.

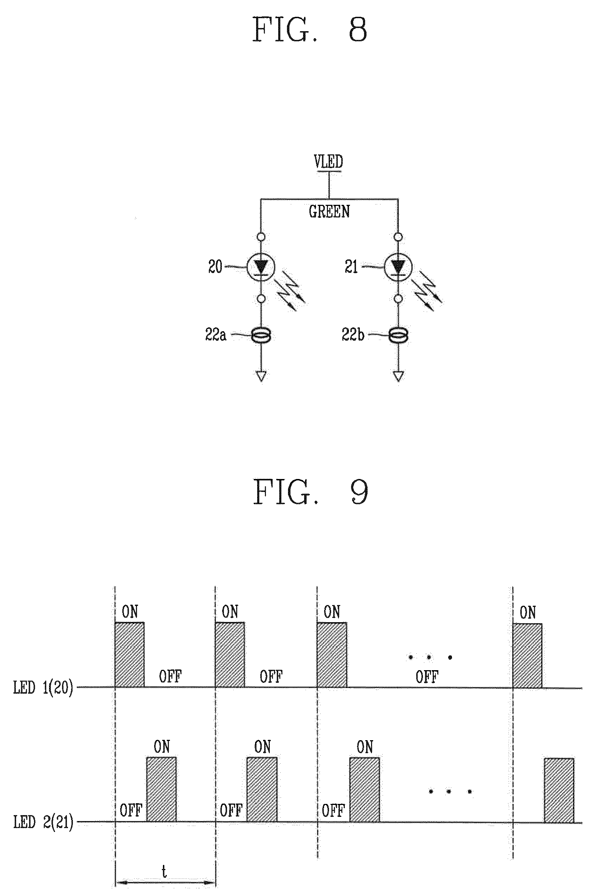

FIG. 8 is a view of a connection structure of two green LEDs of a PPG sensor in accordance with an embodiment of the present invention, and FIG. 9 is a view illustrating an on/off operation of the two green LEDs in accordance with the present invention. As illustrated in FIG. 8, two green LEDs 20 and 21 are connected to LED drivers (or switches) 22a and 22b, respectively. Each of the LED drivers 22a and 22b are devices for turning on/off the green LEDs 20 and 21 and may be configured as a MOS transistor FET.

The LED drivers 22a and 22b can be individually activated in response to driving signals. Hence, when driving signals which are synchronized with each other at the same driving signal period (t) are applied to the LED drivers 22a and 22b, respectively, as illustrated in FIG. 9, the green LEDs 20 and 21 can be turned on/off in an alternating manner. That is, each of the LED drivers 22a and 22b can be turned on at a different time within the same driving signal period (t).

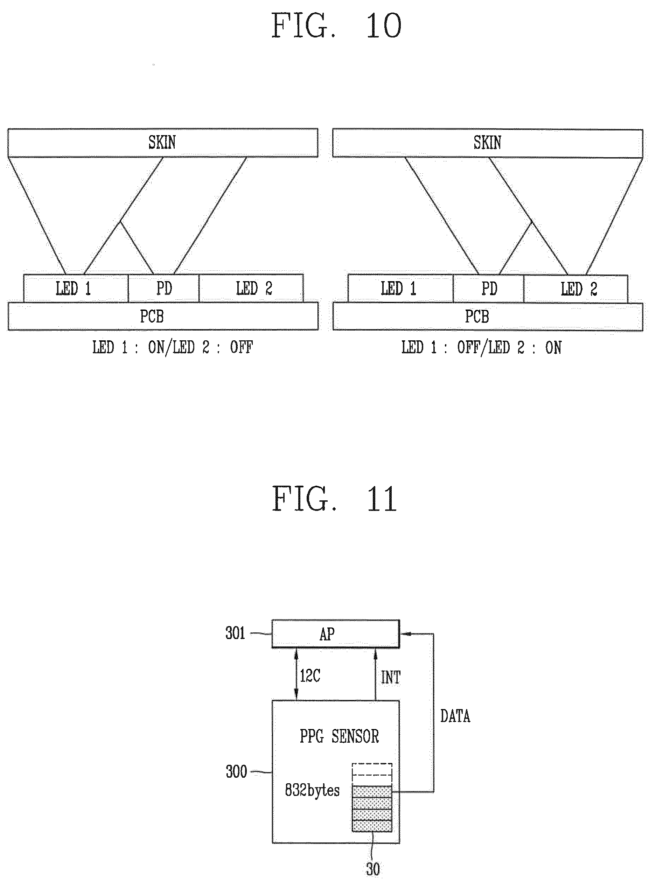

FIG. 10 is a view illustrating an operation of sensing PPG signals by emitting green light from the two green LEDs in accordance with an embodiment of the present invention. As illustrated in FIG. 10, when the two green LEDs 20 and 21 (or LED1 and LED2) are turned on in an alternating manner to emit green light to a user's skin, the photodiode PD can sense green light of the LEDs 20 and 21, reflected on the skin, thereby sensing two PPG signals at one driving signal period (t).

This driving method is advantageous because of less current consumption and higher detection efficiency of the PPG signals, as compared to the method of simultaneously turning on the two green LEDs at the same driving signal period (t) to sense one PPG signal (FIGS. 5A and 5B). Specifically in one embodiment of the present invention, if the LEDs 20 and 21 are turned on/off in the alternating manner at a synchronized timing, only one LED is turned on, which reduces current consumption and ensures a measurement coverage.

Next, FIG. 11 is a view illustrating buffer sharing between a PPG sensor and an AP in accordance with an embodiment of the present invention. As illustrated in FIG. 11, the two green LEDs 20 and 21 (or LED1 and LED2) of the PPG sensor 300 can be turned on in the alternating manner to emit green light to the skin, and the photodiode PD can sense green light of the LEDs 20 and 21, reflected on the skin, respectively, so as to generate respective PPG signals. Each of the PPG signals may include heartbeat data and stress data.

The sensed PPG signals are also stored in the buffer 30. When an amount of PPG signals stacked in the buffer 30 reaches a preset threshold value (e.g., 832 bytes), the PPG sensor 300 can awaken the AP 301 by applying an interrupt signal INT to the AP 301, and thereafter transfer the PPG signals stacked in the buffer 30 to the AP 301 at once.

Accordingly, the AP 301 can measure heartbeat/stress by analyzing qualities of the PPG signals of the two green LEDs 20 and 21, transferred from the PPG sensor 300. The measured heartbeat/stress can also be output on the display unit 151, and stored in the memory 170. Further, one embodiment of the present invention defines the PPG signals of the two green LEDs as first and second channel data, respectively.

In addition, green light emitted from two green LEDs of a PPG sensor are generally absorbed by blood vessels and the remaining green light is reflected so as to generate a PPG signal. Therefore, a performance or quality of the PPG signal can be checked based on a perfusion index (PI) and a signal-to-noise ratio (SNR) of the AP 301. That is, the AP 301 can calculate one perfusion index (PI) by detecting an AC value through a band pass filtering for each sample of the PPG data (signal), and then dividing the detected AC value by a DC value of the PPG data.

Afterwards, the same method can be applied to a plurality of samples to obtain a plurality of PIs. Then, the obtained PIs can be divided by a number of samples N, thereby calculating a final PI. Also, the AP 301 can calculate the SNR by determining 30 bpm to 210 bpm of a frequency region of a received PPG signal as a signal, and the other frequency region as noise.

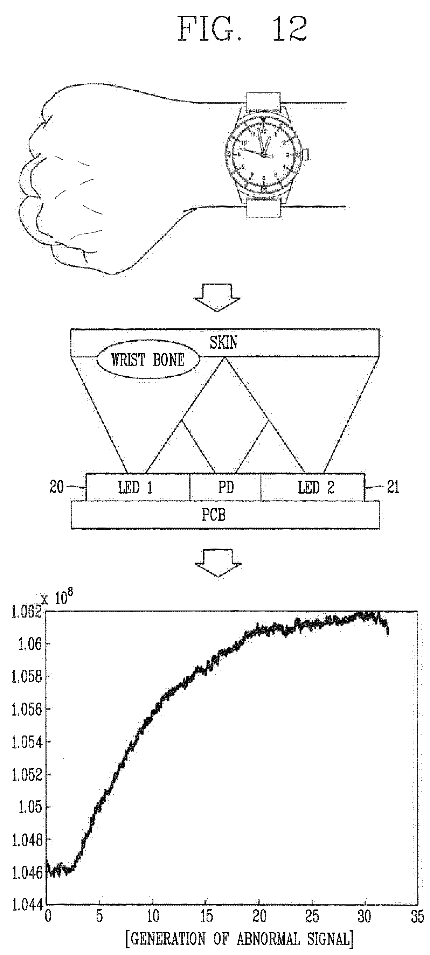

Next, FIG. 12 is a view illustrating one example in which an abnormal signal is generated when measuring heartbeats using a smart watch with the PPG sensor. As illustrated in FIG. 12, when a user wears the smart watch with the PPG sensor on the wrist, a specific LED (e.g., LED1, 20) may be located on a bone which is protruded on the wrist according to a worn shape and position of the smart watch.

In this instance, a PPG signal (PPG signal of a second channel or a second channel PPG signal) which is sensed through the photodiode PD by the green light emitted from an LED 21 may have a normal form, but a PPG signal (PPG signal of a first channel or a first channel PPG signal) which is sensed through the photodiode PD by the green light emitted from the LED 20 may have an abnormal form.

Therefore, in one embodiment of the present invention, after analyzing the signal qualities of the two PPG signals, which are detected when the two green LEDs 20 and 21 are operated in the alternating manner, if a green LED with a PPG signal having a bad quality (from which an abnormal signal is detected) is turned off, the heartbeat can be accurately measured and current consumption can be reduced.

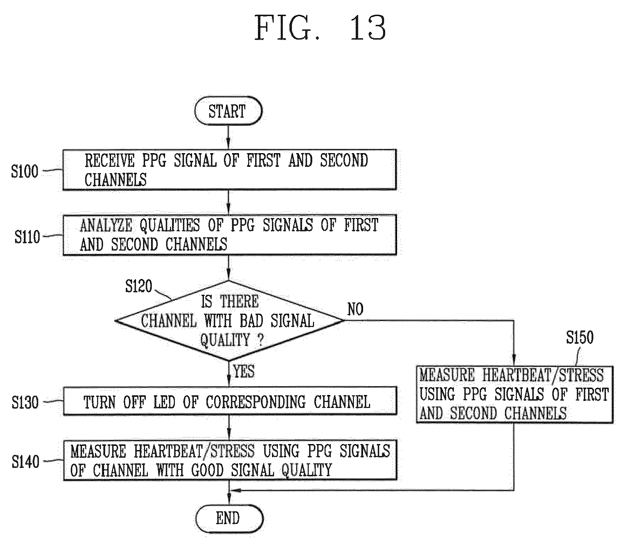

FIG. 13 is a flowchart illustrating adaptively controlling LEDs of a PPG sensor according to qualities of PPG signals in accordance with one embodiment of the present invention. Here, the PPG signals measured by the two green LEDs may be referred to as PPG signals of first and second channels, respectively.

As illustrated in FIG. 13, during a consecutive heartbeat measurement, the AP 301 can receive PPG signals of first and second channels, which are detected by the photodiode PD after the two green LEDs are operated in an alternating manner, from the PPG sensor 300 (S100).

Afterwards, the AP 301 analyzes qualities of the received PPG signals of the first and second channels (S 110), and checks whether or not a channel with bad signal quality (a channel with an abnormal signal generated therefrom) is present (S120). In this instance, an SNR or perfusion index PI may be used as an analysis factor. When there is the channel with the bad signal quality (or channel with the abnormal signal) (Yes in S120), the AP 301 turns off the green LED 20 or 21 of the corresponding channel (S130), and then measures the heartbeat/stress by selecting the PPG signal of the other channel with good signal quality (S140).

Further, if there is no channel with the bad signal quality (or the channel with the abnormal signal) (No in S120), the AP 301 can measure the heartbeat/stress using the PPG signals of the first and second channels (S150). Therefore, the present invention minimizes the current consumption by turning off an LED of a channel which outputs abnormal waveforms during a heartbeat/stress measurement.

Also, because the smart watch is not fixedly worn on a wrist, its worn position can change according to a motion of the wrist. Therefore, because the smart watch is moved in response to the motion of the wrist, the quality of the PPG signal of the first channel or the quality of the PPG signal of the second channel can be poor for a short period of time.

Therefore, when the motion of the wrist is sensed by an acceleration sensor, if the PPG signals are collected by jumping to a channel generating a good signal at each driving period, the PPG signal can be obtained even without turning off an LED of a channel with bad signal quality.

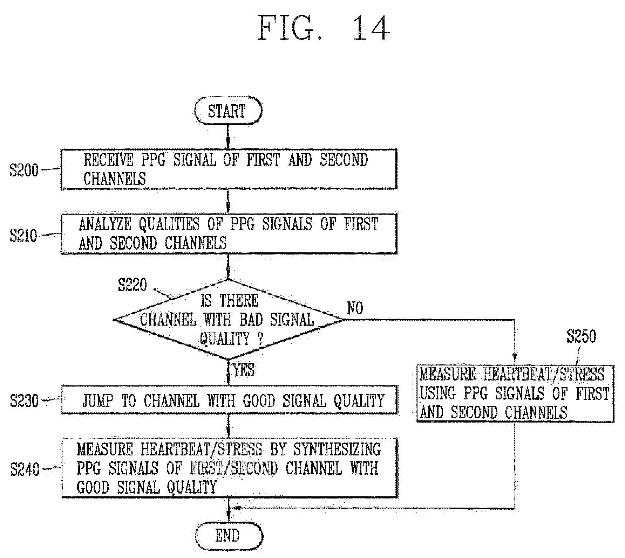

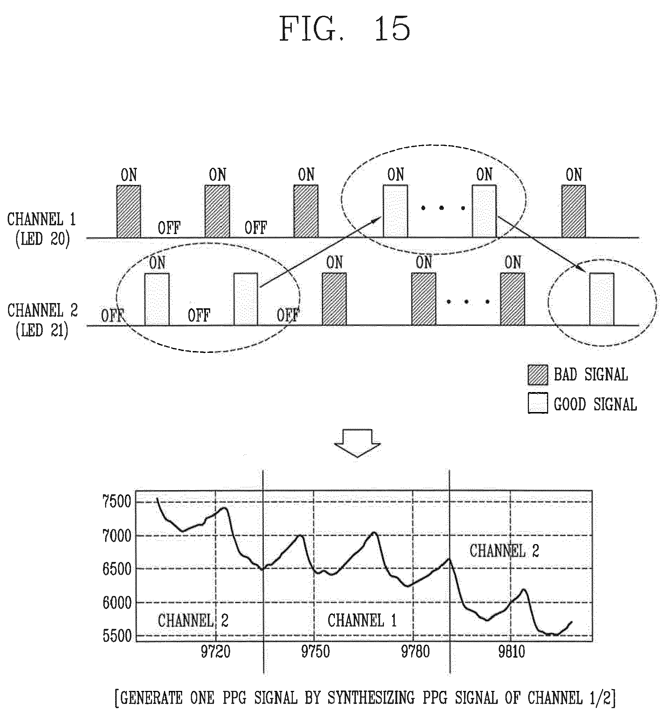

Next, FIG. 14 is a flowchart illustrating adaptively controlling LEDs of a PPG sensor according to qualities of PPG signals in accordance with another embodiment of the present invention, and FIG. 15 is a view illustrating one example of generating one PPG signal by synthesizing (or combing) PPG signals of a first/second channel with bad qualities.

As illustrated in FIG. 14, during a consecutive heartbeat measurement, the AP 301 can receive PPG signals of first and second channels, which are detected by the photodiode after the two green LEDs are operated in the alternating manner, from the PPG sensor 300 (S200).

Afterwards, the AP 301 can analyze qualities of the received PPG signals of the first and second channels (S210), and check whether or not a channel with bad signal quality (a channel with an abnormal channel generated therefrom) is present (S220). In this instance, an SNR or perfusion index PI may be used as an analysis factor.

When there is the channel with the bad signal quality (or channel with the abnormal signal) (Yes in S220), the AP 301, as illustrated in FIG. 15, does not turn off an LED of the channel with the bad signal quality, but jumps to a channel with a high signal quality (channel 1 ↔ channel 2) at each driving period, to select the PPG signals. The AP 301 can synthesize or combine the PPG signals of the selected first/second channel to generate one PPG signal (S230).

Afterwards, the AP 301 can measure heartbeat/stress using the synthesized PPG signal (S240). Further, when there is no channel with the bad signal quality (or the channel with the abnormal signal) (No in S220), the AP 301 can measure the heartbeat/stress using the PPG signals of the first and second channels (S250).

As aforementioned, the PPG signal quality of each channel can depend on the worn position of the smart watch on the wrist and the motion of the smart watch. However, the present invention is not limited to this. That is, the PPG signal quality of each channel may depend on a skin color and an amount of hair on the skin. This is because reflected green light on the skin can differ according to the skin color and the amount of hair and thereby a level of the PPG signal is lowered.

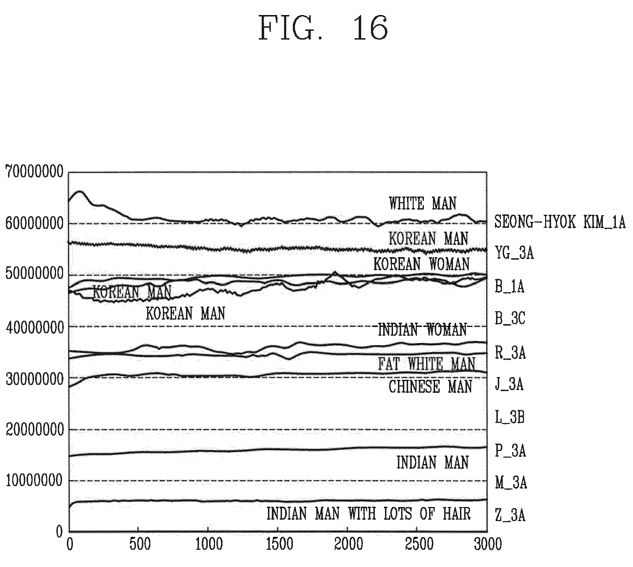

For example, FIG. 16 is a graph illustrating levels of PPG signals according to a skin color and an amount of hair on the skin. As illustrated in FIG. 16, when measuring heartbeats through the PPG sensor while wearing the smart watch on the wrist, a good PPG signal can be detected from a white men (male) with a bright skin color even though a less quantity of green light is emitted from the green LEDs.

Further, a lower level of a PPG signal can be detected from a user who has a dark skin color and a lot of hair on the skin. A better PPG signal can also be detected from a woman (female) with less hair than a man although they have the same skin color.

Therefore, one embodiment of the present invention provides a method of enhancing measurement efficiency of heartbeat/stress by adjusting intensities of green light emitted from green LEDs according to a skin color and an amount of hair on the skin. The intensity of the green light can be adjusted by adjusting strength of current flowing through each green LED.

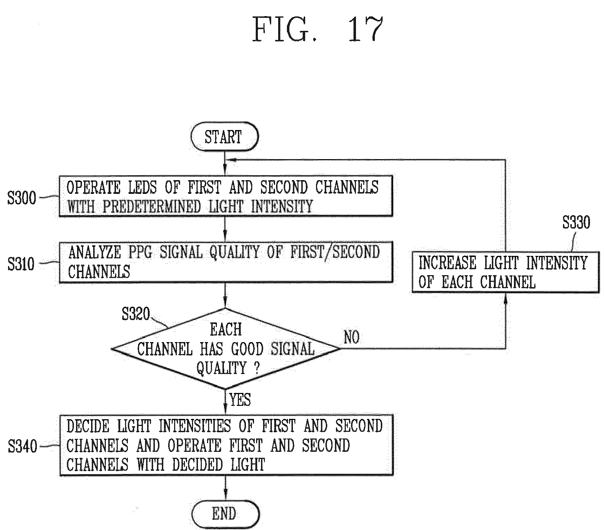

FIG. 17 is a flowchart illustrating adaptively controlling intensities of light emitted from LEDs of a PPG sensor according to a skin color of a wrist and an amount of hair thereon. As illustrated in FIG. 17, when a user wears a smart watch on a wrist to measure their heartbeat/stress, the PPG sensor 300 can detect PPG signals by operating (or driving) green LEDs of first and second channels (two green LEDs) with preset light intensity (current) (S300). The AP 301 can analyze qualities of the first and second channel PPG signals detected in the PPG sensor 300 to check whether or not each channel has a good signal quality (S310 and S320).

When a PPG signal quality (level) of each channel is lower than a preset reference quality (level) (No in S320), the intensity of light emitted from the green LED of each channel can be increased, and those steps S300 to S320 can be repetitively performed.

Afterwards, when the PPG signal quality (level) of each channel is increased higher than the preset reference quality (level) (Yes in S320), the AP 301 can decide the increased light intensity as light intensities of the first and second channels, and then control the PPG sensor 300 to detect the PPG signals by operating the green LEDs of the first and second channels with the decided light intensity (S350).

Accordingly, the present invention can enhance the measurement efficiency of the heartbeat/stress by controlling the green LEDs of the PPG sensor according to the skin color and the amount of hair. Specifically, the operations illustrated in FIG. 17 can be useful for setting light intensity (current) of the green LED of each channel at the beginning of measuring the heartbeat/stress.

In addition, an embodiment of the present invention commonly uses the PPG signal and the PPG data, but this is merely illustrative for the sake of explanation, and their meanings can be construed as the same as each other. The operations in the flowcharts illustrated in FIGS. 13, 14 and 17 may be performed in combination thereof. For example, FIG. 17 may be used at the beginning of the heartbeat/stress measurement, and FIGS. 14 and 17 can be used during the heartbeat/stress measurement.

As aforementioned, the present invention reduces a fabrication cost and an installation area of a heartbeat/stress measuring circuit by employing a simple structure including a PPG sensor and an AP. Also, the heartbeat/stress can be measured with low power, even without employing a low-power MCU, by way of a buffer sharing between the PPG sensor and the AP without frequently waking the AP during the heartbeat/stress measurement.

In addition, the present invention remarkably reduces currents, which are consumed during an operation of a PPG sensor in the related art, by turning on/off the green LEDs of the two channels in an alternating manner at one driving period by independently operating green LEDs of two channels provided in the PPG sensor.

Also, the present invention can turn off an LED of a channel with bad signal quality or selectively use only a PPG signal of a channel with good signal quality, by adjusting intensities of light emitted from two green LEDs of a PPG sensor according to a skin color and an amount of hair prior to a heartbeat/stress measurement, and analyzing PPG signal quality of each channel output from the two green LEDs of the PPG sensor during the heartbeat/stress measurement. This results in reducing current consumption by the LEDs and enhancing accuracy of the heartbeat/stress measurement.

The present invention can be implemented as computer-readable codes in a program-recorded medium. The computer-readable medium may include all types of recording devices each storing data readable by a computer system. Examples of such computer-readable media may include hard disk drive (HDD), solid state disk (SSD), silicon disk drive (SDD), ROM, RAM, CD-ROM, magnetic tape, floppy disk, optical data storage element and the like. Also, the computer-readable medium may also be implemented as a format of carrier wave (e.g., transmission via an Internet). The computer may include the controller 180 of the terminal.

Therefore, it should also be understood that the above-described embodiments are not limited by any of the details of the foregoing description, unless otherwise specified, but rather should be construed broadly within its scope as defined in the appended claims, and therefore all changes and modifications that fall within the metes and bounds of the claims, or equivalents of such metes and bounds are therefore intended to be embraced by the appended claims.

## Claims

1. A circuit in a mobile terminal for measuring heartbeat/stress, the circuit comprising:
a photoplethysmography (PPG) sensor (300) having first and second green light-emitting diodes (LEDs) (20, 21);
first and second LED drivers (22a, 22b) respectively connected to the first and second green LEDS (20, 21) and respectively configured to drive the first and second green LEDs (20, 21); and
a processor (301) configured to:
control the first and second LED drivers (22a, 22b) to alternately turn on the first and second green LEDs (20, 21) within one driving period to respectively produce first and second PPG signals, and
measure a user's heartbeat/stress using the first and second PPG signals that have a corresponding signal quality equal to or greater than a predetermined threshold.

2. The circuit of claim 1, wherein the PPG sensor (300) comprises a first-in, first-out (FIFO) buffer (30) configured to store the first and second PPG signals,
wherein the PPG sensor is further configured to wake up the processor when an amount of the first and second PPG signals stored in the buffer reaches a threshold value.

3. The circuit of anyone of claims 1 and 2, wherein the PPG sensor (300) is further configured to transfer the stored first and second PPG signals to the processor (301) at once when the amount of the first and second PPG signals stored in the buffer reaches the threshold value.

4. The circuit of anyone of claims 1 through 3, wherein the threshold value is set based on a heartbeat output data rate (ODR) of the PPG sensor (300), a stress ODR of the PPG sensor (300), and a number of sensed bytes of the PPG sensor (300).

5. The circuit of anyone of claims 1 through 4, wherein the processor (301) is further configured to adaptively adjust light intensities of the first and second green LEDs (20, 21) by analyzing qualities of the first and second PPG signals according to a user's skin and an amount of hair on the skin,
wherein quality analysis factors of the first and second PPG signals include a perfusion index (PI) and a signal-to-noise ratio (SNR).

6. The circuit of anyone of claims 1 through 5, wherein the processor (301) is further configured to turn off a corresponding green LED having an abnormal signal generated therefrom of the first and second green LEDs (20, 21).

7. The circuit of anyone of claims 1 through 6, wherein the processor (301) is further configured to select and synthesize one of the first and second PPG signals having a corresponding sufficient signal quality at each driving period to measure the user's heartbeat/stress.

8. A circuit in a mobile terminal for measuring heartbeat/stress, the circuit comprising:
a photoplethysmography (PPG) sensor (300) configured to detect PPG signals of first and second channels by operating first and second green light-emitting diodes (LEDs) (20, 21) at each driving period; and
a processor (301) configured to measure a user's heartbeat/stress by analyzing the PPG signals of the first and second channels detected by the PPG sensor (300),
wherein the PPG sensor (300) includes a buffer (30) configured to store the detected PPG signals of the first and second channels, and wake up the processor when an amount of the PPG signals of the first and second channels stored in the buffer reaches a threshold value.

9. The circuit of claim 8, further comprising:
first and second LED drivers (22a, 22b) respectively connected to the first and second green LEDs (20, 21) and respectively configured to drive the first and second green LEDs (20, 21),
wherein the processor (301) is further configured to control the first and second LED drivers (22a, 22b) to respectively turn on the first and second green LEDs (20, 21) in an alternating manner by being synchronized with each other within one driving period.

10. The circuit of anyone of claims 8 and 9, wherein the PPG sensor (300) is further configured to transfer the stored PPG signals to the processor at once when the amount of the PPG signals stored in the buffer reaches a threshold value,
wherein the threshold value is set based on a heartbeat output data rate (ODR) of the PPG sensor, a stress ODR of the PPG sensor, and a number of sensed bytes of the PPG sensor.

11. The circuit of anyone of claims 8 through 10, wherein the processor (301) is further configured to adaptively adjust light intensities of the first and second green LEDs (20, 21) by analyzing qualities of the PPG signals according to a user's skin and an amount of hair on the skin,
wherein quality analysis factors of the PPG signals include a perfusion index (PI) and a signal-to-noise ratio (SNR).

12. The circuit of anyone of claims 8 through 11, wherein the processor (301) is further configured to turn off a corresponding green LED having an abnormal signal generated therefrom of the first and second green LEDs (20, 21).

13. A method of controlling a mobile terminal for measuring heartbeat/stress, the method comprising:
detecting (S100, S110), via a photoplethysmography (PPG) sensor (300) having first and second green light-emitting diodes (LEDs), first and second PPG signals by alternately turning on the first and second green LEDs within one driving period; and
measuring (S150), via a processor (300), a user's heartbeat/stress using the first and second PPG signals that have a corresponding signal quality equal to or greater than a predetermined threshold.

14. The method of claim 13, further comprising:
waking up the processor when an amount of the first and second PPG signals stored in the buffer reaches a threshold value; and
transferring the stored first and second PPG signals to the processor at once when the amount of the first and second PPG signals stored in the buffer reaches the threshold value.

15. The method of claim 13 or 14, wherein the measuring the heartbeat/stress includes:
turning off (S130) a corresponding green LED having an abnormal signal generated therefrom of the first and second green LEDs; and
selecting and synthesizing (S140) one of the first and second PPG signals having a corresponding sufficient signal quality at each driving period to measure the user's heartbeat/stress.
